(19) Europäisches Patentamt
European Patent Office
Office européen des brevets

(11) Publication number: 0 060 123
B1

(12) EUROPEAN PATENT SPECIFICATION

(45) Date of publication of patent specification: 29.10.86

(51) Int. Cl.[4]: G 01 N 33/542, C 12 Q 1/42, C 12 Q 1/32

(21) Application number: 82301170.5

(22) Date of filing: 08.03.82

# (54) Assay and use.

(30) Priority: 07.03.81 GB 8107249

(43) Date of publication of application: 15.09.82 Bulletin 82/37

(45) Publication of the grant of the patent: 29.10.86 Bulletin 86/44

(84) Designated Contracting States: BE CH DE FR GB IT LI NL SE

(56) References cited:
EP-A-0 027 036
EP-A-0 032 286
WO-A-80/02747
GB-A-2 034 466
US-A-3 730 844

CHEMICAL ABSTRACTS, vol. 72, no. 9, 2nd March 1970, page 163, no. 40547b, Columbus Ohio (USA); J.W. HEKKELMAN et al.: "Possible role of alkaline phosphatase in the chain of actions of parathyroid hormone on bone cell metabolism".
J. Biol.Chem.236,2746-2755 (1961)

(73) Proprietor: Self, Colin Henry
46 Lensfield Road
Cambridge, CB2 1EG (GB)

(72) Inventor: Self, Colin Henry
46 Lensfield Road
Cambridge, CB2 1EG (GB)

(74) Representative: McCall, John Douglas et al
W.P. THOMPSON & CO. Coopers Building
Church Street
Liverpool L1 3AB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method of determining a conjugate between a ligand or receptor and a phosphatase which uses the ability of the phosphatase in the conjugate to dephosphorylate NADP and thereby start a cyclic chemical reaction and to the use of the method. More particularly this invention relates to an assay in which a NAD/NADH cycle is employed and to the use of that assay.

In my earlier European Patent Application EP—A—0027036 I disclosed inter alia how a ligand or receptor could be determined by carrying out an assay requiring a conjugate between a ligand or receptor and a primary enzyme which was capable of producing a modulator for a secondary system, allowing the primary system to produce the modulator and allowing the secondary system to function in the presence of the modulator and determining a product of that secondary system. Since operation of the secondary system continues once it has been switched on by the primary system considerable amplification occurred in my aforementioned application which employed a NAD/NADH inter-conversion and NAD acted as modulator for the secondary system. A particularly suitable method of utilising this secondary system for the detection of a ligand or receptor has now been devised and employs a conjugate between the ligand or receptor and a phosphatase.

The use of pyridine nucleotide cycles has been known for many years since the original work of O. H. Lowry et al., (J. Biol. Chem., 236: 2746—2755 (1961)). Such cycles have enabled amplification of signals and so have been used to aid in the measurement of small amounts of materials. However, they have not been employed to measure enzyme conjugate used, for example, in immunoassays. In part this is because such systems to date have generally needed to use severe reaction conditions to remove the residual precursor from which the pyridine nucleotide is produced or which produces a material which first has to be converted into a different chemical entity in order to start an amplification cycle. Phosphatase has been used to dephosphorylate NADP (see J. W. Hekkelman et al., Chem Abst., 72, 40547b (1970)) although conjugates of phosphatases have not been used in this way. I have now discovered that conjugates of phosphatases may be employed as labels in assays which employ a phosphate of a nicotinamide adenine dinucleotide as substrate for the conjugates and by using the resulting nicotinamide adenine dinucleotide itself to start a cyclic chemical reaction, not only are the inconveniences of the prior art avoided, a highly sensitive assay system results.

One aspect of this invention provides a method for determining a ligand or receptor which method comprises carrying out an assay for the ligand or receptor, the assay requiring a labelled component wherein the labelled component is a conjugate between a ligand or receptor and a phosphatase capable of producing NAD which is a modulator for a secondary system which interconverts NAD and NADH and determining that portion of labelled component to be determined by allowing the conjugate between the ligand or receptor and phosphatase to produce NAD, allowing the secondary system to function in the presence of the NAD, and determining a product of the secondary system.

Since, as in my aforesaid application, production of NAD by the primary system switches on the secondary system, considerable amplification is achievable since operation of the secondary system produces an increasing chemical change as the cycle keeps turning.

According to the present invention there is provided a method of determining a conjugate between a ligand or receptor and a phosphatase wherein the enzymic activity of the conjugate leads to a detectable change which is detected which comprises contacting a source of said conjugate with a phosphate of a nicotinamide adenine dinucleotide whereby said phosphate of a nicotinamide adenine dinucleotide is dephosphorylated to a nicotinamide adenine dinucleotide which starts a cyclic chemical reaction which interconverts said nicotinamide adenine dinucleotide and its reduced form and which produces a detectable change which is detected.

From the foregoing it will be appreciated that an aspect of this invention comprises a method of determining a conjugate between a ligand or receptor and a phosphatase which method comprises contacting a source of said conjugate with NADP whereby the NADP is dephosphorylated to NAD which starts a cyclic chemical reaction which interconverts NAD and NADH and produces a detectable change which is detected.

When used herein the term "starts" means that the presence of the material allows a cycle to occur which would not otherwise occur.

Since this invention relies on the production of NAD (the modulator for the secondary system) by the action of a conjugate of a phosphatase, the skilled worker will appreciate that the assay will be carried out in the presence of NADP. Schematically therefore the latter part of the assay may be represented thus:

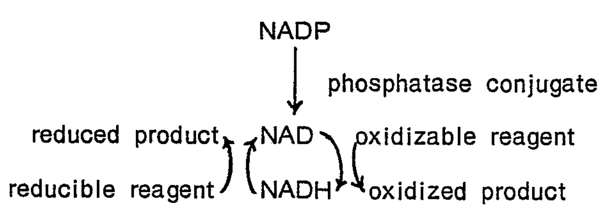

Either the oxidized product or the reduced product may be determined during the assay. I believe that one of the considerable advantages of my amplified assay is that it allows for the detection of a product by eye or by the use of simple optical measuring devices. One particularly suitable method of producing an optically detectable colour change is to employ MTT tetrazolium (also known as thiazolyl blue or 3-(3,4-dimethylthiazolyl-2)-2,5-diphenyltetrazolium) which on reduction can change from a yellow colour to a dark colour (for example blueish/greyish/blackish colour). Thus a favoured reducible reagent for use is MTT tetrazolium. This reagent often produces a more marked colour change in the presence of an electron transfer reagent such as PES (also known as phenazine ethosulphate).

The skilled worker will appreciate that this form of the invention may be adapted to the detection of phosphatase conjugates such as those of acid or alkaline phosphatase. The skilled worker will appreciate that the detection of such conjugates is useful in conventional assays for materials which have affinity for the ligand or receptor. Since the classes of compounds which have affinities for each other are antibodies and antigens, these are the preferred ligands and receptors for use in the conjugates. The use of such conjugates in enzyme immunoassay systems such as ELISA and EMIT systems is well known. The use of conjugates of antibodies and antigens in such systems is well known and in general conjugates of alkaline phosphatase, especially with antibodies, are particularly useful.

The change detected as a result of carrying out the method of this invention may be the removal of a reagent consumed by the operation cycle or it may be the production of a product produced by operation of the cycle. Preferably the change detected as a result of carrying out the method of this invention is due to the production of a product produced by operation of the cycle. The change detected as a result of carrying out the method of this invention may

(a) be the removal of a reagent directly consumed by operation of the cycle, or

(b) be the appearance of a product directly produced by operation of the cycle, or

(c) be indirectly caused by the intermediacy of other reactions. Preferably the change detected is due to the direct removal or more preferably to the direct production of material.

The reagents employed will be chosen to produce a detectable change which is more preferably a change visible to the eye, for example the production or removal of colour. Preferably the reagents are chosen so that at least one visibly coloured product is formed.

It has been found that by employing a reagent which may be oxidized or reduced as a result of operation of the cycle a readily detectable change may be made to occur. Most aptly therefor a reagent is employed which on oxidation or reduction is converted to a product which is visually detectable.

Normally the conversion of the oxidisable reagent to the oxidized product is enzymatically catalysed. The enzyme employed is favourably a dehydrogenase.

A suitable oxidisable reagent for use in the NADP/NADPH cycle is isocitrate (for example as the sodium salt) which is converted to α-ketoglutarate (the oxidised product) in the presence of NADP-specific isocitrate dihydrogenase.

A suitable reducible reagent for use with NADPH/NADP cycle is oxidised glutathione which in the presence of glutathione reductase produces reduced glutathione which may be detected by its reaction with 5,5'-Dithio-bis-(2-nitrobenzoic acid) known as DTNB.

A suitable oxidisable reagent for use in the NAD/NADH cycle is lactate (for example the sodium salt) which is converted to pyruvate (the oxidised product) in the presence of lactate dehydrogenase. A very favoured oxidisable reagent for use in the NAD/NADH cycle is ethanol which is converted to acetaldehyde (the oxidised product) in the presence of alcohol dehydrogenase.

Other oxidizable reagents which may be oxidized by their appropriate dehydrogenase include glyoxalate, acetaldehyde, D-glycerate, L-malate, dihydro-orotic acid, α-glycerophosphate, triosephosphate, D-glucose, lipoamide, lipoate, glutathione, L-β-hydroxybutyryl CoA, uridinediphosphoglucuronic acid, β-hydroxysteroids such as 3-α-hydroxysteroids and 17β-hydroxysteroids.

A desirable reducible reagent for use in the NAD/NADH cycle is thiazolyl blue (also known as MTT or 3-(4,5-dimethylthiazol-2-yl)-2,5-diphenyl-tetrazolium bromide) which is converted to a reduced, coloured readily detectable product. Tetrazolium salts which produce a colour change on reduction by NAD/NADH cycle are a favoured class of reducible agents. This reduction is enhanced by an electron transfer reagent such as phenazine ethosulphate (also known as PES) or phenazine methosulphate. A preferred electron transfer reagent is phenazine ethosulphate. The reduction product of the thiazolyl blue produced during the operation of the method of this invention can be detected by the eye or monitored at 570nm, if desired, in a conventional manner.

The use of the above reagents in a favoured form of this invention may be represented schematically thus:

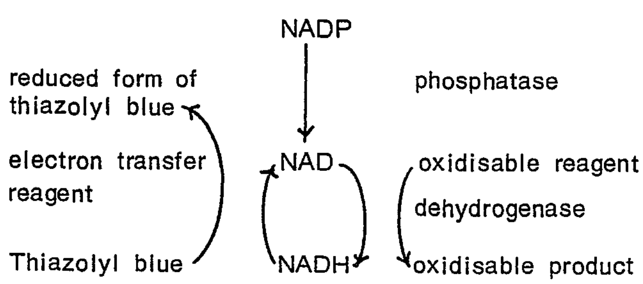

The skilled worker will relise that cycles of the type envisaged herein will be carried out in aqueous solution and most suitably under mild conditions; that is at non-extreme temperatures such as 1° to 40°C and more generally 5° to 38°C and at a pH of from 5 to 10 and more usually 5.5 to 9.3.

A favoured oxidizable reagent for use in the NAD/NADH cycle is an alcohol such as ethanol which can be oxidized by alcohol dehydrogenase into an oxidized product such as acetaldehyde.

From the foregoing the skilled worker will appreciate that a more detailed representation of the preceding schematic representation may be written thus:

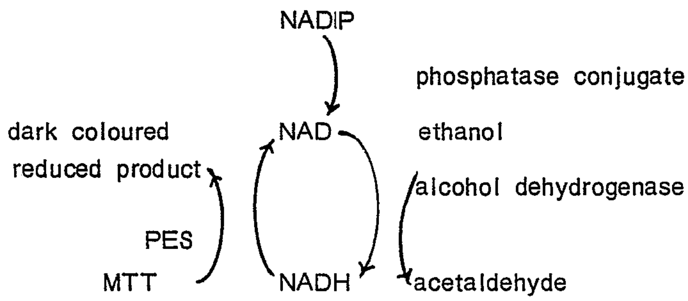


Once the preceding primed cycle is switched on by the production of NAD by the primary cycle, a colour change can be detected (by eye or machine). Clearly the system will utilize NAD-free reagents since the modulator should not be introduced except by the action of the phosphatase conjugate on the NADP.

The phosphatase conjugate may be from any convenient source. The phosphatase may be of the alkaline type or the acid type. The phosphatase may be bound to a ligand or receptor using any convenient method such as by reaction with a bifunctional conjugating reagent such as SPDP (N-succinimidyl-2-(2-pyridyldithio)propionate).

The skilled worker will appreciate that such methods of conjugating enzymes are well known in the art. Most desirably the phosphatase is conjugated to an antibody or an antigen. Preferably, the phosphatase is conjugated to an antibody. The antibody may be an antibody against any antigen including those instances where the antigen is another antibody.

From another aspect, this invention provides a method of determining a conjugate between a ligand or receptor and a phosphatase which method comprises contacting said conjugate with NADP and the components of a NAD/NADH cycle other than NAD or NADH and determining a product of that cycle.

Most suitably the ligand and receptor are an antibody or antigen although other moieties are envisaged, for example, a drug and its receptor.

From another view, this invention provides a method of determining a conjugate between an antibody or antigen and a phosphatase which method comprises contacting said conjugate with NADP and the components of a NAD/NADH cycle other than NAD and NADH and determining a product of that cycle.

Naturally the method will be carried out under conditions such that when NAD is produced by the conjugate the cycle will operate. Since sufficient amounts of the reagents required to drive the cycle will be present an amplification is achieved that greatly increases the sensitivity of the detection method. Normally, reagents employed are in sufficient excess so that their concentrations do not limit the amplification achievable.

Most aptly the conjugate employed is a conjugate of alkaline phosphatase since it has been discovered that the optimal pH for the cyclic reactions is very suitable for the operation of alkaline phosphatase (pH 8—10.5, more aptly 9—9.5 for example 9.3). This allows one to cause the primary reaction and the cycle to proceed simultaneously if desired. If a conjugate of acid phosphatase is used the initial reaction is generally carried out at pH 4—6, for example 5.6. At this pH the cycle does not work efficiently so that when the pH is raised to allow the cycle to operate the acid phosphatase is effectively switched off and produces no further NAD. This has the advantage that simpler kinetics and easier quantification can be achieved.

Alkaline pH values as outlined above may be achieved using conventional buffers such as an ethanolamine/HCl buffer. Acid pH values as outlined above may also be achieved using conventional buffers such as citrate buffer (buffers of both types may be obtained from commercial suppliers such as Sigma).

The assays of this invention may be carried out at any non-extreme temperature such as 5—45°C but generally it is preferred to carry out the assay at ambient temperatures.

Most desirably this invention provides a method of amplifying an enzyme linked immunosorbent assay (ELISA) system which utilizes a phosphatase linked to a ligand or receptor wherein the amplification is achieved by using the phosphatase linked to a ligand or receptor to produce NAD from NADP which NAD starts a NAD/NADH cycle one product of which is determinable.

The reagents and products of this amplified system may be as hereinfore described.

ELISA systems which may be amplified by this invention include those adapted to the detection of: Malaria; Amoebiasis; Schistomosiasis; Onchocerciasis; Toxoplasmosis; Hydatidosis; Trichinella; Babesia; Leishmaniasis; Trypanosome; Cytomegalovirus; Hepatitis B antigen; Measles; Rubella; Plant Viruses;

Erythrocyte Antigens; Factor Viii-related antigens; antibodies against rubella, cholera, E. coli; Salmonella O antigen; markers of oncological importance such as alpha-foeto-protein; hormones such as thyroid hormones and sex hormones; baculoviruses; gentamicin.

ELISA systems are well known — see for example: Voller, A. & Bidwell, D. E. (1975) Brit. J. Exp. Path. *56*:338

Engvall, E & Perlman, P. (1971) Immunochem. 8:871 Voller, A., Bidwell, D. Huldt, G. & Engvall, E. (1974) Bull. Wld. Hlth. Org. *51*:209

Carlsson, H. E., Lindberg, A. A. & Hammerstein, S. (1972) Infect. Immun. *6*:703

Voller, A., Bidwell, J. E. & Bartlett, A (1976) Microplate Enzyme Immunoassays of Virus Infections — from Manuals of Clinical Immunology, Chapt. 69 (Ed. Rose, N. & Friedman, H.), Am. Soc. Microbiol. p506.

Veldkamp, J. & Visser, A. M. (1975) Brit. J. Vener. Dis. *51*:227

Voller, A., Bidwell, D. E. & Bartlett, A. (1976) Bull. Wld. Hlth. Org. *53*:55.

Voller, A., Bidwell, D. E. and Bartlett, A. (1976) the Application of Micro-Plate Enzyme-Linked Immunosorbent Assays to some Infectious Diseases in the First International Symposium on Immunoenzymatic Techniques INSERM Symposium No. 2 (Ed. Feldman et al) North Holland Publishing Co.

The method of this invention may be used to diagnose disease by detecting disease markers in human fluids, for example blood, serum, urine, tissue homogenates which are removed from the body.

Purified antibody against the antigen to be detected is associated with a solid surface, by methods known in the art. The sample solution to be detected is brought into contact with the surface for a determined time (such as half a minute to two hours) under mild conditions of temperature (such as 4°C to 40°C) and pH (such as 5.0 to 8.0) with possible mild agitation. The solution is taken off and the solid surface washed free of remaining unbound material. Another antibody reactive with the antigen (which may be initially the same or different to the previous antibody) but which has been conjugated to phosphatase and maintained soluble is then brought into contact with the solid for a determined time (such as half a minute to two hours) under mild conditions of temperature (such as 4°C to 40°C) and pH (such as 5.0 to 8.0) with possible mild agitation. The solution is taken off and the solid surface washed free of unbound material. The determination of the remaining solid-associated phosphatase is then conducted as follows: NADP at a suitable concentration (such as 0.5mM to 2.0mM) is brought into contact with the solid surface in a buffer suitable for any solid-associated phosphatase to dephosphorylate it to NAD (for alkaline phosphatase buffers buffering in the range of such as pH 7.8 to 10 and usually 8.8 to 9.5) and in the presence of magnesium ion (at a concentration such as 1mM to 5mM). Incubation is carried out (for half a minute to 2 hours) at a fixed temperature from 4°C to 40°C. The amount of NAD formed is then determined by a cyclic assay for example by the addition of materials which will reduce NAD for example an enzyme and its substrate (such as NAD-dependent alcohol dehydrogenase, a suitable amount being from 4 to 40 units of enzyme activity) and ethanol (from 3mM to 1.2mM) and materials which will oxidise the NADH so formed for example PES and MTT). Either the reduction or oxidation of NAD or NADH is followed as a result of an irreversible event taking place during the reaction, and in the above example this can be the development of a dark blue-brown colour as the MTT is reduced. This may be determined by eye or measured at 570nm.

An extremely effective ELISA system is presently available as Rubelisa® Test Kit from M. A. Bioproducts, Walkersville, Maryland 21793, USA. This Test Kit (their catalogue number 30—3000) is a sensitive method for the determination of Rubella virus IgG antibody in human serum. However the method recommends a relatively long final incubation period and the use of a spectrophotometer. Amplification of this test by the method of this invention allows for the reduction of the incubation period and allows visual determination (without the necessary use of a spectrophotometer).

References are made to the Rubelisa® Test Kit manual and to the European Application EP — A — 0058539.

The following example illustrates this invention:

Using a Rubelisa® Test Kit —

A Rubelisa® 96-well plate is taken and labelled as necessary. The wells are washed by filling each with PBS—Tween® from a wash bottle and then air bubbles are removed from the wells by moving the plate gently back and forth. The PBS-Tween is shaken from the wells into a disposal receptacle containing a solution of 5% household bleach and each well refilled with PBS-Tween and the air bubbles removed again. The plate is then left for three minutes and then emptied again as above. The whole process is repeated two more times — the final time ensuring complete emptying by tapping the upside-down plate on a clean paper towel. Reconstituated serum diluent (250µl) is then added to each well. The patients' individual sera are shaken to homogeneity and then 5µl of each serum is added to one well containing rubella antigen and also to one well containing control antigen. Serum is withdrawn and expelled three times in the well to help mix it. It is of utmost importance to use different pipettes for different sera. Three control sera (negative, low positive and high positive) are included with each test that is carried out. Three pairs of wells must therefore be used for these controls. If less than three patients' sera are to be tested at any one time it is advised that these can be positioned immediately following the test sera on the plate. However, if the plate is used to its maximum capacity of 45 separate test sera then it is advised that the control samples are distributed on the plate as: high positive — A1 & B1; low positive — C11 & D11; negative — G12 & H12 (5 µl of each). If fewer than 45 patients' sera are tested it is advisable to place them with the high positive at the beginning, the low positive in the middle and the negative at the end of the

batch of sera tested. After all of the sera are added the plate is placed on a Micromixer for a few minutes. The plate is placed in a plastic bag with a wet cotton or paper pledget, the bag sealed to allow a humid atmosphere to develop and kept at room temperature (20—25°) for two hours. The plastic bag is then opened and the liquids are shaken from their wells into the disposal receptacle. The wells are then washed again as described above (with PBS-Tween®, four times). Then 250 µl of 50-fold diluted conjugated antiserum supplied with the kit is added to each well. The plate is incubated as above in the humid bag for a further 2 hours at room temperature, after which the liquid is shaken from the wells into the disposal receptacle and the plate washed again four times as above. The wells are now ready to be assayed for their alkaline phosphatese content. To each well is added 210 µl 0.14M ethanolamine-HCl buffer at pH 9.3 and containing 5mM $MgCl_2$. The following are added to each well: 10 µl 10mM thiazolyl blue (MTT), 10 µl of 40mM phenazine ethosulphate (PES), 5 µl of alcohol dehydrogenase supplied by the Sigma Chemical Company Ltd especially free of NAD (catalogue number A3263) and 5 µl of 1M ethanol. NADP is then added to each well. This is done by adding 10 µl of a 10mM solution of NADP to each well. Depending on the number of samples being tested and the accuracy required this may be achieved either by adding the solution to each successive well quickly and then mixing on a Micromixer or (with many samples) by adding the solution at fixed intervals (such as a second to minutes) from one well to the next. The reactions are then monitored by observing the colour change of the solutions from pale yellow towards black visually — taking account of any differences in time of addition of the NADP solutions to the various wells.

The solution in the well initially coated with rubella antigen and to which high positive control serum was added will change colour at a much faster rate than the solution in a rubella antigen well to which serum containing significantly less anti-rubella antibody activity was added, and also much faster than the well containing control antigen to which the high positive control serum was added.

If required, the reaction bringing about the colour change can be stopped, after a suitable period (such as minutes to hours — depending on the sensitivity required) for example 5 minutes, by for example changing the pH of the solution. To achieve this the contents of a well can be removed to a vessel, such as a 1.0ml colorimeter cuvette, containing 0.75 ml of 2M citrate buffer at pH 4.8, the solutions mixed and the degree of previous reaction documented by measuring the degree of colour change from pale yellow to black against a standard chart or by use of a colorimeter set to 570nm. This alternative has the advantage that the degree of reaction does not have to be documented at the very time it is occurring.

In another alternative, the wells may be assayed for their alkaline phosphatase content by allowing them to first act on a standard NADP solution which is then transferred to another vessel for the colour generating reaction to take place. In this alternative, the NAD concentration is not therefore continuously increasing whilst the colour reaction is taking place and so quantification is somewhat easier. For this, 250 µl of 0.14M ethanolamine buffer at pH 9.3 and containing 5mM $MgCl_2$ and 0.4mM NADP is added to each well to be assayed. The plate is incubated at room temperature for a time such as minutes to hours (depending on the sensitivity required) for example 5 minutes. At the end of this period the contents of each well are added to suitable vessels containing 10 µl of 10mM MTT, 10 µl of 40mM PES, 5 µl of alcohol dehydrogenase (A3263), 5 µl of 1M ethanol in 0.14M ethanolamine buffer at pH 9.3 sufficient to make the final volume 1.0ml, and the contents mixed. Again the colour change of the solutions from pale yellow to black is monitored either visually or with a colorimeter set to 570nm. The results of these alternative approaches will be found to be in accordance with that stated above i.e. wells initially containing rubella antigen which receive a serum high in anti-rubella antibody, by the method of the test, will give rise to a faster development of black colour and wells which do not meet these criteria.

## Claims

1. A method of determining a conjugate between a ligand or receptor and a phosphatase wherein the enzymic activity of the conjugate leads to a detectable change which is detected characterised in that a source of said conjugate is contacted with a phosphate of nicotinamide adenine dinucleotide whereby said phosphate of a nicotinamide adenine dinucleotide is dephosphorylated to a nicotinamide adenine dinucleotide which starts a cyclic chemical reaction which interconverts said nicotinamide adenine dinucleotide and its reduced form and which produces a detectable change which is detected.

2. A method as claimed in claim 1 wherein a conjugate between a ligand or receptor and an acid phosphatase is contacted with NADP which is dephosphorylated to NAD which starts a cyclic chemical reaction which interconverts NAD and NADH and produces a detectable change which is detected.

3. A method as claimed in claim 1 wherein a conjugate between a ligand or receptor and an alkaline phosphatase is contacted with NADP which is dephosphorylated to NAD which starts a cyclic chemical reaction which interconverts NAD and NADH and produces a detectable change which is detected.

4. A method as claimed in claim 1 for determining a ligand or receptor which method comprises carrying out an assay for the ligand or receptor, the assay requiring a labelled component wherein the labelled component is a conjugate between a ligand or receptor and a phosphatase capable of producing NAD which is a modulator for a secondary system which interconverts NAD and NADH and determining that portion of labelled component to be determined by allowing the conjugate between the ligand or receptor and phosphatase to produce NAD, allowing the secondary system to function in the presence of the NAD, and determining a product of the secondary system.

5. A method as claimed in any one of claims 2 to 4 wherein the detectable change is a visually detectable change produced by the oxidation or reduction of an oxidizable or reducible substrate as a result of operation of the cycle.

6. A method as claimed in claim 5 wherein the colour change is produced by reduction of a tetrazolium salt.

7. A method as claimed in any of claims 2 to 6 wherein the conjugate is a conjugate between a phosphatase and an antigen or antibody.

8. A method as claimed in any of claims 3 to 6 wherein the conjugate is a conjugate between an alkaline phosphatase and an antibody or antigen.

9. A method as claimed in any of claims 2 to 8 wherein ethanol is used as a reagent for the cyclic chemical reaction which interconverts NAD and NADH, which ethanol is oxidized by alcohol dehydrogenase by operation of the cyclic chemical reaction.

10. A method as claimed in either of claims 8 or 9 carried out at a pH of 8 to 10.5.

11. A method as claimed in any of claims 2 to 10 which is carried out at ambient temperature.

12. A method as claimed in claim 1 of amplifying an enzyme linked immunosorbent assay which utilizes a phosphatase linked to a ligand or receptor wherein the amplification is achieved by using the phosphatase linked to a ligand or receptor to produce NAD from NADP which NAD starts a NAD/NADP cycle, a product of which is determined.

13. A method as claimed in claim 1 of determining a conjugate between a ligand or receptor and a phosphatase which method comprises contacting said conjugate with NADP and the components of a NAD/NADH cycle other than NAD or NADH and determining a product of that cycle.

14. A method as claimed in either of claims 12 or 13 wherein the conjugate is an alkaline phosphatase conjugated to an antigen.

15. A method as claimed in either of claims 12 or 13 wherein the conjugate is an alkaline phosphatase conjugated to an antibody.

16. A method as claimed in any of claims 12 to 15 wherein the detectable change is a visually detectable change which results from the production of an oxidized product or reduced product of the cyclic chemical reaction.

17. A method as claimed in either of claims 15 or 16 carried out at a pH of 8 to 10.5.

18. A method as claimed in claim 17 which is carried out at ambient temperature.

19. A method of diagnosing disease which comprises using a method as claimed in any of claims 2 to 18 to determine a disease marker on a sample of human fluid outside the human body.

20. A method as claimed in claim 19 wherein the human fluid is serum.

21. A method as claimed in either of claims 19 or 20 wherein the disease is hepatitis B.

22. A method as claimed in either of claims 19 or 20 wherein the disease marker is a thyroid hormone.

23. A method as claimed in any one of claims 8 to 22 for the detection of an antigen, wherein an antibody is associated with a solid surface and is used to bind the antigen to be detected, causing the bound antigen to react with a phosphatase labelled antibody, and determining the enzyme activity by adding NADP as a substrate to start the cyclic reaction.

24. A kit for carrying out the method of claim 23 comprising:—

1. a first antibody against the antigen to be detected, immobilised on a solid surface;

2. a second antibody against the antigen to be detected, conjugated to phosphatase, said second antibody being the same as or different from said first antibody;

3. NADP;

4. a material susceptible either to oxidation or reduction in said method, to produce a detectable colour change.

**Patentansprüche**

1. Verfahren zur Bestimmung eines Konjugats zwischen einem Liganden oder Rezeptor und einer Phosphatase, wobei die Enzymaktivität des Konjugats zu einer nachweisbaren Änderung führt, die nachgewiesen wird, dadurch gekennzeichnet, daß eine Quelle dieses Konjugats mit einem Phosphat eines Nicotinamidadenindinucleotids kontaktiert wird, wodurch dieses Phosphat eines Nicotinamidadenindinucleotids zu einem Nicotinamidadenindinucleotid dephosphoryliert wird, das eine cyclische chemische Reaktion in Gang setzt, durch die das Nicotinamidadenindinucleotid und seine reduzierte Form ineinander umgewandelt werden und durch die es zu einer nachweisbaren Änderung kommt, die nachgewiesen wird.

2. Verfahren nach Anspruch 1, bei dem ein Konjugat zwischen einem Liganden oder Rezeptor und einer sauren Phosphatase mit NADP kontaktiert wird, das zu NAD desphosphoryliert wird, das eine cyclische chemische Reaktion in Gang setzt, durch die NAD und NADH ineinander umgewandelt werden und durch die es zu einer nachweisbaren Änderung kommt, die nachgewiesen wird.

3. Verfahren nach Anspruch 1, bei dem ein Konjugat zwischen einem Liganden oder Rezeptor und einer alkalischen Phosphatase mit NADP kontaktiert wird, das zu NAD dephosphoryliert wird, das eine cyclische chemische Reaktion in Gang setzt, durch die NAD und NADH ineinander umgewandelt werden und durch die es zu einer nachweisbaren Änderung kommt, die nachgewiesen wird.

4. Verfahren nach Anspruch 1 zur Bestimmung eines Liganden oder Rezeptors, wobei man in diesem

Verfahren zur Bestimmung des Liganden oder des Rezeptors einen Versuch durchführt, der eine markierte Komponente erfordert, wobei diese ein Konjugat zwischen einem Liganden oder Rezeptor und einer Phosphatase ist, die NAD zur erzeugen vermag, das ein Modulator für ein sekundäres System ist, durch das NAD und NADH ineinander übergeführt werden, und den Teil der zu bestimmenden markierten Komponente bestimmt, indem man das Konjugat zwischen dem Liganden oder Rezeptor und der Phosphatase NAD erzeugen läßt, das sekundäre System in Anwesenheit der NAD arbeiten läßt, und ein Produkt des sekundären Systems ermittelt.

5. Verfahren nach einem der Ansprüche 2 bis 4, bei dem die nachweisbare Änderung eine visuell nachweisbare Änderung ist, hervorgerufen durch die Oxydation oder Reduktion eines oxydierbaren oder reduzierbaren Substrats als Ergebnis des Reaktionscyclus.

6. Verfahren nach Anspruch 5, bei dem der Farbumsclag durch Reduktion eines Tetrazoliumsalzes erzeugt wird.

7. Verfahren nach einem der Ansprüche 2 bis 6, bei dem das Konjugat ein Konjugat zwischen einer Phosphatase und einem Antigen oder einem Antikörper ist.

8. Verfahren nach einem der Ansprüche 3 bis 6, bei dem das Konjugat ein Konjugat zwischen einer alkalischen Phosphatase und einem Antikörper oder einem Antigen ist.

9. Verfahren nach einem der Ansprüche 2 bis 8, bei dem als Reagens für die cyclische chemische Reaktion, durch die NAD und NADH ineinander übergeführt werden, Ethanol verwendet wird, das durch eine Alkoholdehydrogenase durch die cyclische chemische Reaktion oxydiert wird.

10. Verfahren nach einem der Ansprüche 8 oder 9, wobei das Verfahren bei einem pH von 8 bis 10,5 durchgeführt wird.

11. Verfahren nach einem der Ansprüche 2 bis 10, das bei Umgebungstemperatur durchgeführt wird.

12. Verfahren nach Anspruch 1 zur Erweiterung eines enzymgebundenen Immunoabsorbensversuchs, bei dem eine an einen Liganden oder Rezeptor gebundene Phosphatase verwendet wird, und bei dem die Erweiterung durch Verwendung der an einen Liganden oder Rezeptor gebundenen Phosphatase zur Erzeugung von NAD aus NADP durchgeführt wird, wobei das NAD einen NAD/NADP-Cyclus initiiert, dessen Produkt ermittelt wird.

13. Verfahren nach Anspruch 1 zur Ermittlung eines Konjugats zwischen einem Liganden oder Rezeptor und einer Phosphatase, wobei in diesem Verfahren das Konjugat mit NADP und den von NAD oder NADH unterschiedlichen Komponenten eines NAD/NADH-Cyclus kontaktiert und ein Produkt dieses Cyclus ermittelt wird.

14. Verfahren nach einem der Ansprüche 12 oder 13, wobei das Konjugat eine mit einem Antigen konjugierte alkalische Phosphatase ist.

15. Verfahren nach einem der Ansprüche 12 oder 13, bei dem das Konjugat eine mit einem Antikörper konjugierte alkalische Phosphatase ist.

16. Verfahren nach einem der Ansprüche 12 bis 15, bei dem die nachweisbare Änderung eine visuell nachweisbare Änderung ist, die sich aus der Erzeugung eines oxydierten oder reduzierten Produktes der cyclischen chemischen Reaktion ergibt.

17. Verfahren nach einem der Ansprüche 15 oder 16, das bei einem pH von 8 bis 10,5 durchgeführt wird.

18. Verfahren nach Anspruch 17, das bei Umgebungstemperatur durchgeführt wird.

19. Verfahren zur Diagnose einer Erkrankung, bei dem man sich zur Bestimmung eines Krankheitsmarkers an einer Probe der menschlichen Flüssigkeit außerhalb des menschlichen Körpers eines Verfahrens nach Anspruch 2 bis 18 bedient.

20. Verfahren nach Anspruch 19, bei dem die menschliche Flüssigkeit Serum ist.

21. Verfahren nach einem der Ansprüche 19 oder 20, bei dem die Krankheit Hepatitis B ist.

22. Verfahren nach einem der Ansprüche 19 oder 20, bei dem der Krankheitsmarker ein Thyroidhormon ist.

23. Verfahren nach einem der Ansprüche 8 bis 22 zum Nachweis eines Antigens, bei dem ein Antikörper mit einer festen Oberfläche verbunden und zur Bindung des nachzuweisenden Antigens verwendet wird, wobei man das gebundene Antigen zur Umsetzung mit einem phosphatasemarkierten Antikörper veranlaßt und die Enzymaktivität durch Zugabe von NADP als Substrat zur Initierung der cyclische Reaktion ermittelt.

24. Satz zur Durchführung des Verfahrens nach Anspruch 23, enthaltend

1) einen ersten Antikörper gegen das nachzuweisende, auf einer festen Oberfläche immobilisierte Antigen,

2) einen zweiten Antikörper gegen das nachzuweisende, mit Phosphatase konjugierte Antigen, wobei der zweite Antikörper mit dem ersten identisch oder von ihm verschieden ist,

3) NADP und

4) einen entweder für die Oxydation oder Reduktion nach dem genannten Verfahren geeigneten Stoff zur Erzeugung eines nachweisbaren Farbumschlags.

**Revendications**

1. Méthode pour déterminer un conjugué entre un ligand ou un récepteur et une phosphatase, dans

laquelle l'activité enzymatique du conjugué conduit à un changement détectable qui est détectée, caractérisée en ce qu'une source de ce conjugué est mise en contact avec un phosphate de nicotinamide adénine dinucléotide de telle sorte que le phosphate de nicotinamide adénine dinucléotide est déphosphorylé en un nicotinamide adénine dinucléotide qui amorce une réaction chimique cyclique qui interchange ce nicotinamide adénine dinucléotide et sa forme réduite et qui produit un changement détectable qui est détecté.

2. Méthode selon la revendication 1, dans laquelle un conjugué entre un ligand ou un récepteur et une phosphatase acide est mis en contact avec le NADP qui est déphosphorylé en NAD, lequel amorce une réaction chimique cyclique qui interchange NAD et NADH et produit un changement détectable qui est détecté.

3. Méthode selon la revendication 1, dans laquelle un conjugué entre un ligand ou un récepteur et une phosphatase alcaline est mis en contact avec le NADP qui est déphosphorylé en NAD lequel amorce une réaction chimique cyclique qui interchange NAD et NADH et produit un changement détectable qui est détecté.

4. Méthode selon la revendication 1 pour déterminer un ligand ou un récepteur, selon laquelle on effectue un essai pour le ligand ou le récepteur, l'essai nécessitant un composant marqué dans lequel le composant marqué est un conjugué entre un ligand ou un récepteur et une phosphatase capable de produire un NAD qui est un modulator pour un système secondaire qui interchange le NAD et la NADH, et l'on détermine cette portion de composant marqué devant être déterminé en permettant au conjugué entre le ligand ou le récepteur et la phosphatase de produire du NAD, en laissant le système secondaire fonctionner en présence du NAD, et en déterminant un produit du système secondaire.

5. Méthode selon l'une quelconque des revendications 2 à 4, dans laquelle le changement détectable est un changement détectable visuellement, produit par l'oxydation ou la réduction d'un substrat oxydable ou réductible à la suite du fonctionnement du cycle.

6. Méthode selon la revendication 5, dans laquelle le changement de couleur est produit par la réduction d'un sel de tétrazolium.

7. Méthode selon l'une quelconque des revendications 2 à 6, dans laquelle le conjugué est un conjugué entre une phosphatase et un antigène ou un anticorps.

8. Méthode selon l'une quelconque des revendications 3 à 6, dans laquelle le conjugué est un conjugué entre une phosphatase alcaline et un antigène ou un anticorps.

9. Méthode selon l'une quelconque des revendications 2 à 8, dans laquelle l'éthanol est utilisé comme réactif pour la réaction chimique cyclique qui interchange le NAD et le NADH, lequel éthanol est oxydé par une alcool déshydrogénase au cours du fonctionnement de la réaction chimique cyclique.

10. Méthode selon l'une quelconque des revendications 8 ou 9, effectuée à un pH 8 à 10,5.

11. Méthode selon l'une quelconque des revendications 2 à 10, qui est effectuée à la température ambiante.

12. Méthode selon la revendication 1 pour amplifier un essai ELISA qui utilise une phosphatase liée à un ligand ou un récepteur, dans laquelle l'amplification est obtenue à l'aide de la phosphatase liée à un ligand ou un récepteur qui produit un NAD à partir de NADP, lequel NAD amorce un cycle NAD/NADP, dont un produit est déterminé.

13. Méthode selon la revendication 1 pour déterminer un conjugué entre un ligand ou un récepteur et une phosphatase, laquelle méthode comprend la mise en contact de ce conjugué avec du NADP et les composants d'un cycle NAD/NADH autres que NAD ou NADH et la détermination d'un produit de ce cycle.

14. Méthode selon l'une des revendications 12 ou 13, dans laquelle le conjugué est une phosphate alcaline conjuguée à un antigène.

15. Méthode selon l'une quelconque des revendications 12 ou 13, dans laquelle le conjugué est une phosphatase alcaline conjuguée à un anticorps.

16. Méthode selon l'une quelconque des revendications 12 à 15, dans laquelle le changement détectable est un changement détectable visuellement, qui résulte de la production d'un produit oxydé ou d'un produit réduit de la réaction chimique cyclique.

17. Méthode selon l'une quelconque des revendications 15 ou 16, effectuée à un pH de 8 à 10,5.

18. Méthode selon la revendication 17, qui est effectuée à une température ambiante.

19. Méthode pour diagnostiquer une maladie, qui comprend l'utilisation d'une méthode selon l'une quelconque des revendications 2 à 18 pour déterminer un marqueur de maladie sur un échantillon d'un fluide humain à l'extérieur du corps humain.

20. Méthode selon la revendication 19, dans laquelle le fluide humain est du sérum.

21. Méthode selon l'une quelconque des revendications 19 ou 20, dans laquelle la maladie est l'hépatite B.

22. Méthode selon l'une quelconque des revendications 19 ou 20, dans laquelle le marqueur de maladie est une hormone de la thyroïde.

23. Méthode selon l'une quelconque des revendications 8 à 22, pour la détection d'un antigène, dans laquelle on associe un anticorps à une surface solide et on l'utilise pour lier l'antigène devant être détecté, on provoque la réaction de l'antigène lié avec un anticorps marqué par une phosphatase, et l'on détermine l'activité enzymatique en ajoutant du NADP comme substrat pour amorcer la réaction cyclique.

24. Kit pour mettre en oeuvre la méthode de la revendication 23, comprenent:

1. un premier anticorps vis-à-vis de l'antigène devant être détecté, immobilisé sur une surface solide;

2. un second anticorps vis-à-vis de l'antigène devant être détecté, conjugué à une phosphatase, ce second anticorps étant identique au premier anticorps ou différent de celui-ci;

3. un NADP;

4. une matière susceptible d'être oxydée ou réduite dans cette méthode, pour produire un changement de couleur détectable.